# EUROPÄISCHE PATENTSCHRIFT

(11) **EP 2 630 976 B1**
(45) Veröffentlichungstag und Bekanntmachung des Hinweises auf die Patenterteilung: **20.09.2017**
(21) Anmeldenummer: 12008512.1
(22) Anmeldetag: 21.12.2012
(51) Int. Cl.: A61L 24/00, A61L 24/06

(54) **Pastenförmiger Knochenzement**
Bone cement in paste form
Ciment osseux malléable

(30) Priorität: 30.01.2012 DE 102012001637
(43) Veröffentlichungstag der Anmeldung: 28.08.2013
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- DE-A1-102010 024 653
- DE-A1-102010 055 759
- PEDERSEN CJ: "CYCLIC POLYETHERS AND THEIR COMPLEXES WITH METAL SALTS", JOURNAL OF THE AMERICAN CHEMICAL SOCIETY, AMERICAN CHEMICAL SOCIETY, US, Bd. 89, 1. Januar 1967 (1967-01-01), Seiten 7017-7036, XP009079172, ISSN: 0002-7863, DOI: 10.1021/JA01002A035

## Beschreibung

Die vorliegende Erfindung betrifft einen Kit, die Verwendung eines Kits zur Herstellung einer Paste zur Herstellung von Spacern oder zur Herstellung von Trägermaterialien für die lokale Antibiotika-Therapie, ein Initiatorsystem, eine polymerisierbare Zusammensetzung sowie einen Formkörper.

Konventionelle Polymethylmethacrylat-Knochenzemente (PMMA-Knochenze-mente) sind seit Jahrzehnten bekannt und gehen auf die grundlegenden Arbeiten von Sir Charnley zurück (Charnley, J.: "Anchorage of the femoral head prosthesis of the shaft of the femur"; J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, (ii) einen Röntgenopaker und (iii) einen Initiator (zum Beispiel) Dibenzoylperoxid, Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen, Ferner muss das Vermischen der Komponenten zügig erfolgen. Dabei kommt es darauf an, dass das gesamte Zementpulver ohne Klumpenbildung mit der Monomerkomponente verwischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4,015,945, EP-A-0 674 888 und JP 2003-181270 offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Als Alternative zu den konventionellen Pulver-Flüssigkeits-Polymethylmethacrylatknochenzementen wurden pastenförmige Polymethylmethacrylatknochen-zemente in den Offenlegungsschriften DE-A-10 2007 052116, DE-A-10 2007 050 762 und DE-A-10 2007 050 763 beschrieben. Dabei werden die Knochenzemente als vorgemischte, lagerstabile Pasten dem Anwender zur Verfügung gestellt. Diese Pasten enthalten jeweils ein radikalisch polymerisierbares Methacrylatmonomer, ein in diesem Methacrylatmonomer lösliches Polymer und ein in diesem Methacrylatmonomer unlösliches partikuläres Polymer (da beide Pasten ein unlösliches partikuläres Polymer beinhalten, werden solche Systeme als "symmetrisch" bezeichnet). In einer dieser Pasten ist zudem ein radikalischer Polymerisationsinitiator enthalten, während die andere Paste einen Polymerisationsaktivator aufweist. Der aus diesen Pasten hergestellte Knochenzement weist als Folge der gewählten Zusammensetzung eine ausreichend hohe Viskosität und Kohäsion auf, um dem Blutungsdruck bis zur Aushärtung standzuhalten. Beim Vermischen der beiden Pasten reagiert der Polymerisationsinitiator mit dem Akzelerator unter Bildung von Radikalen, die die radikalische Polymerisation der Methacrylatmonomere initiieren.

Das bei den bisherigen aus einer Pulverkomponente und einer Monomer-flüssigkeit bestehenden PMMA-Knochenzementen eingesetzte Initiatorsystem Dibenzoylperoxid und N,N-Dxmethyl-p-toluidin hat sich prinzipiell bewährt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Das Dibenzoylperoxid liegt dabei als Feststoff im Zementpulver vor und das N,N-Dimethyl-p-toluidin ist in der Monomerkomponente gelöst.

Eigene Versuche mit Zementpasten unter Verwendung des Initiatorsystems Dibenzoylperoxid/N,N-Dimethyl-p-toluidin zeigten allerdings, dass Pasten mit N,N-Dimethyl-p-toluidin eine ausgeprägte Tendenz zur Spontanpolymerisation besitzen. Weiterhin wird der bei konventionellen Pulver/Flüssigkeits-Polymethyl-methacrylat-Knochenzementen bewährte Beschleuniger N,N-Dimethyl-p-toluidin toxikologisch kritisch diskutiert.

Neben diesem Redoxsystem wurden auch Initiatorsysteme beschrieben, die auf der Verwendung von Barbituraten beruhen. So beschreiben DE -A-10 2007 050 762 und DE -A-10 2007 050 763 ein Initiatorsystem umfassend Erdalkalisalze von Barbitursäurederivaten, Halogenid-Ionendonatoren und Kupferverbindungen. Dabei sind Erdalkalisalze von Barbituraten und basische Kupfersalze in einer Paste enthalten. Diese beiden Salze sind im Methacrylat-Monomeren unlöslich. In einer zweiten Paste ist eine schwache organische Säure, wie 2-Ethylhexansäure, enthalten, Daneben ist noch ein Chlorid-Ionendonator in den Pasten vorhanden, wobei gemäß der Lehre der DE -A-10 2007 050 763 vorzugsweise Tetraalkylammoniumchlorid als Chlorid-Ionendonator eingesetzt wird. Bei Vermischung der beiden Pasten wird durch die schwache organische Säure synchron sowohl das Barbiturat in die lösliche Säureform als auch das Kupfer in ein lösliches Kupfersalz überführt. Der Vorteil dieses Systems ist insbesondere bei Pasten mit mehrfachfunktionellen Monomeren, dass durch vorgelagerte Diffusions- und Ionenaustauschprozesse eine Erhöhung der Verarbeitungszeit möglich ist, die sonst bei der Verwendung von mehrfach funktionelle Monomeren nur sehr kurz ist und üblicherweise im Sekundenbereich liegt. Nachteilig ist jedoch, dass mitunter quartäre Ammoniumchloride in Gegenwart von gelösten Schwermetallsalzen Spontanpolymerisationen auslösen können. Es hat sich daher als vorteilhaft erwiesen, ein Initiatorsystem auf Basis von Barbituraten, Schwermetall-Ionen und Chlorid-Ionen zu entwickeln, wobei die Chlorid-Ionen in Form von anorganischen Salzen anstelle der aus dem Stand der Technik bekannten Tetraalkylammoniumchloride einzusetzen. Ein solches Initiatorsystem wird beispielsweise in der DE 10 2010 024 653 A1 beschrieben. Natriumchlorid, Kaliumchlorid und Kalziumchlorid lösen sich im üblichen, für Knochenzemente verwendeten hydrophoben Monomeren, wie Methylmethacrylat, nacht. Lithiumchlorid löst sich etwas in Methylmethacrylat. Jedoch kann es bei Anwesenheit von Wasserspuren zu schwer reproduzierbaren Veränderungen des Initiationsverhaltens kommen.

Der vorliegenden Erfindung lag die Aufgabe zugrunde, die sich aus dem Stand der Technik ergebenden Nachteile im Zusammenhang mit auf mindestens zwei Pasten basierenden Knochenzement-Systemen zu überwinden.

Insbesondere lag der vorliegenden Erfindung die Aufgabe zugrunde, einen Kit auf der Basis zweier Pasten bereitzustellen, wobei sich die Pasten, solange sie getrennt voneinander vorliegen, durch eine möglichst hohe Polymerisationsstabilität auszeichnen (also möglichst nicht zur Spontanpolymerisation neigen).

Auch lag der vorliegenden Erfindung die Aufgabe zugrunde, einen Kit auf der Basis zweier Pasten bzw. ein Initiatorsystem bereitzustellen, bei dem bei dem Alkalichloride und auch Erdalkalichloride auch in Gegenwart von Wasserspuren sicher in hydrophoben Methacrylatmonomeren, wie Methylmethacrylat, gelöst werden können. Der Kit sollte sich durch ein möglichst reproduzierbares Initiationsverhalten auszeichnen.

Einen Beitrag zur Lösung der eingangs genannten Aufgaben leistet ein Kit, aufweisend eine Paste A und eine Paste B,
wobei
(a) Paste A
   (a1) wenigstens ein radikalisch polymerisierbares Monomer, und
   (a2) als Polymerisationsinitiator wenigstens ein Barbitursäurederivat beinhaltet;
(b) Paste B
   (b1) wenigstens ein radikalisch polymerisierbares Monomer,
   (b2) als Polymerisationsbeschleuniger wenigstens eine Schwermetallverbindung, die aus der Gruppe bestehend aus Schwermetallsalzen und Schwermetalllcomplexen ausgewählt ist,
   (b4) wenigstens ein Alkali- oder Erdalkalihalogenid, und
   (b5) wenigstens einen Komplexbildner für die Alkali-Ionen oder Erdalkali-Ionen (b4), der wenigstens zwei Ethergruppen enthält, beinhaltet;
und wobei mindestens eine der Pasten A und B als Komponente (a3) bzw. (b3) wenigstens einen in (a1) bzw. (b1) unlöslichen Füllstoff beinhaltet.

Die Erfindung basiert unter anderem auf der Idee, eine erste Paste zu verwenden, die ein in einem radikalisch polymerisierbaren Monomer, wie Methylmethacrylat, lösliches Barbiturat enthält. Bei Vermischung dieser ersten Paste mit einer zweiten Paste, welche neben dem radikalisch polymerisierbaren Monomer eine Schwermetallverbindung, eine Alkali- oder Erdalkalihalogenid und einen Komplexbildner für die Alkali-Ionen oder Erdalkali-Ionen enthält, reagiert das lösliche Barbiturat auf Grund seiner Acidität mit dem vorzugsweise basischen Schwermetallsalz. Überraschend zeigte sich, dass dadurch die Polymerisationsreaktion in Gegenwart eines anorganischen, vorzugsweise in dem Monomer löslichen Halogenidionen-Donators initiiert werden kann. Durch Einwirkung des Barbiturats auf das vorzugsweise basische Schwermetallsalz werden die Schwermetall-Ionen offensichtlich in eine lösliche Salzform überführt, welche durch Einwirkung auf die Barbitursäure die Polymerisation des Methacrylatmonomeren initiiert. Durch den Einsatz der Komplexbildner-Komponente (b5) in der Paste B wird sichergestellt, dass sich unabhängig vom Wassergehalt in der Paste B stets ausreichende Mengen des Alkali- Erdalkalihalogenids (n4) im radikalisch polymerisierbaren Monomer (b1) der Paste B lösen, wodurch ein reproduzierbares Initiationsverhalten sichergestellt wird.

Erfindungsgemäß wird unter Kit ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Paste A beinhaltet als Komponente (a1) ein radikalisch polymerisierbares Monomer, wobei es sich vorzugsweise um ein Monomer handelt, welches bei einer Temperatur von 25°C und einem Druck von 1,013 hPa flüssig ist.

Das radikalisch polymerisierbare Monomer (a1) ist vorzugsweise ein Methacrylat-Monomer, insbesondere ein Methacrylsäureester. Vorzugsweise handelt es sich bei dem Methacrylsäureester (a1) um einen monofunktionellen Methacrylsäureester. Dieser ist vorzugsweise hydrophob. Durch die Verwendung von hydrophoben monofunktionellen Methacrylsäureestern (a1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem radikalisch polymerisierbaren Monomer (a1) um Methacrylsäuremethylester, Methacrylsäureethylester oder um eine Mischung aus diesen beiden Monomeren.

Gemäß einer weiteren bevorzugten Ausführungsform handelt es sich bei dem radikalisch polymerisierbaren Monomer (a1) nicht um einen von Bisphenol A abgeleiteten Methacrylsäureester.

Das erfindungsgemäß eingesetzte radikalisch polymerisierbare Monomer (a1) weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer (a1) zeichnet sich vorzugsweise dadurch aus, dass eine wässrige Lösung des radikalisch polymerisierbaren Monomers (a1) einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Paste A enthält vorzugsweise 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste A, des wenigstens einen radikalisch polymerisierbaren Monomers (a1).

Paste A beinhaltet weiterhin als Komponente a2) wenigstens ein Barbitursäurederivat als Polymerisationsinitiator, wobei es sich bei diesem Barbitursäurederivat vorzugsweise um ein Barbitursäurederivat ausgehwählt aus der Gruppe bestehend aus 1-monosunbstituierten Barbituraten, 5-monosubstituierten Barbituraten, 1,5-disubstituierten Barbituraten, 1,3,5-trisubstituierten Barbituraten und 1,3,5-tetrasubstituierten Barbituraten handelt, Unter diesen besonders bevorzugt sind diejenigen, welche die Blut-Hirn-Schranke nicht oder allenfalls in nicht pharmakologisch relevanter Menge zu überwinden vermögen. In diesem Zusammenhang ganz besonders bevorzugte Barbitursäurederivate sind daher 1-monosunbstituierte Barbiturate, 5-monosubstituierte Barbiturate, 1,5-disubstituierte Barbiturate und 1,3,5-trisubstituierte Barbiturate, wobei 1,5-disubstituierte Barbiturate und 1,3,5-trisubtituierte Barbiturate am meisten bevorzugt sind.

Gemäß einer bevorzugten Ausführungsform ist das Barbitursäurederivat (a2) in dem polymerisierbaren Monomer (a1) löslich. Das Barbitursäurederivat (a2) ist in dem polymerisierbaren Monomer (a1) löslich, wenn sich das Barbitursäurederivat (a3) in dem polymerisierbaren Monomer (a1) zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löst.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein.

Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf.

Erfindungsgemäß bevorzugt sind Barbiturate, die an Position 1 und an Position 5 jeweils einen Substituenten, an den Positionen 1, 3 und 5 jeweils einen Substituenten oder an den Positionen 1 und 3 jeweils einen Substituenten und an Position 5 zwei Substituenten tragen.

Gemäß einer besonders bevorzugten Ausfürhungsform ist das Barbitursäurederivat (a2) aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Die Paste A beinhaltet das wenigstens eine Barbitursäurederivat (a2) vorzugsweise in einer Menge ein einem Bereich von 0,1 bis 10 Gew.-%, mehr bevorzugt in einem Bereich von 0,5 bis 8 Gew.-% und noch mehr bevorzugt in einem Bereich von 1 bis 5 Gew.-%, jeweils bezogen auf das Gesamtgewicht der Paste A.

Paste B beinhaltet als Komponente (b1) ebenfalls ein radikalisch polymerisierbares Monomer, wobei es sich vorzugsweise um ein Monomer handelt, welches bei einer Temperatur von 25°C und einem Druck von 1.013 hPa flüssig ist. Das radikalisch polymerisierbare Monomer (b1) kann in einem Kit identisch sein von dem radikalisch polymerisierbaren Monomer (a1) oder sich von diesem unterscheiden, wobei es bevorzugt ist, dass das radikalisch polymerisierbare Monomer (a1) und das radikalisch polymerisierbare Monomer (b1) identisch sind.

Das radikalisch polymerisierbare Monomer (b1) ist vorzugsweise ein Methacrylat-Monomer, insbesondere ein Methacrylsäureester. Vorzugsweise handelt es sich bei dem Methacrylsäureester (b1) um einen monofunktionellen Methacrylsäureester. Dieser ist vorzugsweise hydrophob. Durch die Verwendung von hydrophoben monofunktionellen Methacrylsäureestern (b1) kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen. Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Gemäß einer besonders bevorzugten Ausführungsform handelt es sich bei dem radikalisch polymerisierbaren Monomer (b1) um Methacrylsäuremethylester, Methacrylsäureethylester oder um eine Mischung aus diesen beiden Monomeren.

Gemäß einer weiteren bevorzugten Ausführungsform handelt es sich bei dem radikalisch polymerisierbaren Monomer (b1) nicht um einen von Bisphenol A abgeleiteten Methacrylsäureester.

Das erfindungsgemäß eingesetzte radikalisch polymerisierbare Monomer (b1) weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer (b1) zeichnet sich vorzugsweise dadurch aus, dass eine wässrige Lösung des radikalisch polymerisierbaren Monomers (b1) einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Paste B enthält vorzugsweise 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste B, des wenigstens einen radikalisch polymerisierbaren Monomers (b1).

Paste B beinhaltet weiterhin als Komponente (b2) wenigstens Schwermetallverbindung, die aus der Gruppe bestehend aus Schwermetallsalzen und Schwermetallkomplexen ausgewählt ist, als Polymerisationsbeschleuniger, wobei es sich als besonders vorteilhaft erwiesen hat, wenn die wenigstens eine Schermetallverbindung (b2) in dem radikalisch polymerisierbaren Monomer (b1) schwer-, vorzugsweise sogar unlöslich ist, Als in dem radikalisch polymerisierbaren Monomer (b1) schwer- oder unlöslich gilt eine Schwermetallverbindung (b2), wenn sich weniger als 1 g/l, vorzugsweise weniger als 0,1 g/l, noch mehr bevorzugt weniger als 0,01 g/l, noch mehr bevorzugt weniger als 0,001 g/l, darüber hinaus bevorzugt weniger als 0,0001 g/l um am meisten bevorzugt überhaupt keine nennenswerten Mengen der Schwermetallverbindung (b2) bei einer Temperatur von 25°C in dem radikalisch polymerisierbaren Monomer (b1) lösen (die Schwermetallverbindung (b2) als in dem radikalisch polymerisierbaren Monomer (b1) unlöslich ist).

Unter Schwermetallverbindung werden erfindungsgemäß Verbindungen von Metallen verstanden, die eine Dichte bei einer Temperatur von 20°C von wenigstens 3,5, vorzugsweise von wenigstens 5 aufweisen.

Gemäß einer bevorzugten Ausführungsform handelt es sich bei der Schwermetallverbindung (b2) um eine basische Schwermetallverbindung. Unter basischer Schwermetallverbindung wird eine Schwermetallverbindung verstanden, die in Wasser gelöst oder suspendiert einen pH-Wert von wenigstens 6,5, bevorzugt wenigstens 7 und noch mehr bevorzugt wenigstens 7,5 aufweist.

Gemäß einer ganz besonders bevorzugten Ausführungsform handelt es sich bei den Schwermetallverbindungen (b2) um Verbindungen von Metallen, die ihre Oxidationsstufe wechseln können. In diesem Zusammenhang erfindungsgemäß bevorzugt sind Verbindungen von Kupfer(II), Eisen (II), Eisen(III), Mangan(II), Mangan(III), Cobalt(II) und Cobalt(III), wobei Kupfer(II)-Verbindungen ganz besonders bevorzugt sind.

Die erfindungsgemäßen Schwermetallverbindungen sind, sofern es sich um in dem radikalisch polymerisierbaren Monomer (b1) schwer- oder unlösliche Schermetallverbindungen handelt, vorzugsweise in der Lage, sich in Gegenwart und der Barbitursäurederivate (a2) in eine in dem radikalisch polymerisierbaren Monomer (a1) bzw. (b1) lösliche Form umzuwandeln.

Erfindungsgemäß handelt es sich bei den Schwermetallverbindungen (b2) um Schwermetallsalze oder Schwermetallkomplexe.

Bei den Schwermetallsalzen (b2) handelt es sich vorzugsweise um Halogenide, Hydroxyde, Carbonate oder Carbonsäuxesalze von Schwermetallen. Bevorzugte Schwermetallsalze sind Salze von Kupfer(II), Eisen(II), Eisen(III), Mangan(II), Mangan(III), Cobalt(II) und Cobalt(III).

Als in (b1) unlösliche Schwermetallverbindung (b2) kommen weiterein Halogenidsalze in Betracht. Das Halogenidsalz kann vorzugsweise aus der Gruppe ausgewählt sein, die aus Chloriden und Bromiden von Schwermetallen besteht. Gemäß einer besonderen Ausführungsform handelt es sich bei dem Halogenidsalz um eine Verbindung, die aus der Gruppe ausgewählt ist, die aus Mangan(II)chlorid, Eisen(II)chlorid, Eisen(III)chlorid, Kobalt(II)chlorid und Kobalt(III)chlorid, besteht.

Gemäß einer besonders bevorzugten Ausführungsform wird das Schwermetallsalz (b2) aus der Gruppe ausgewählt, die aus Kupfer(II)hydroxid, basischem Kupfer(II)carbonat oder einer Mischung aus mindestens zwei davon, insbesondere einer Mischung Kupfer(II)hydroxid und basischem Kupfer(II)carbonat, besteht.

Die Paste B beinhaltet die Schwermetallverbindung (b2) vorzugsweise in einer Menge in einem Bereich von 0,0005 bis 0,5 Gew.-%, mehr bevorzugt in einem Bereich von 0,001 bis 0,05 Gew.-% und ganz besonders bevorzugt in einem Bereich von 0,001 bis 0,01 Grew.-%, jeweils bezogen auf das Gesamtgewicht der Paste B.

Paste B beinhaltet weiterhin als Komponente (b4) wenigstens ein Alkali- oder Erdalkalihalogenid. Als Halogenid-Anion kommen grundsätzlich F", Cl⁻ und Br⁻ in Betracht, wobei Cl⁻ ganz besonders bevorzugt ist. Zu den besonders bevorzugten Alkali- oder Erdalkalihalogeniden gehören Kaliumchlorid, Natriumchlorid, Kalziumchlorid und Magnesiumchlorid, wobei Lithiumchlorid als Alkalichlorid (b4) am meisten bevorzugt ist.

In diesem Zusammenhang ist es weiterhin bevorzugt, dass die Paste B das mindestens eine Alkali- oder Erdalkalihalogenid (b4) in einer Menge in einem Bereich von 0,001 bis 7,5 Gew.-%, besonders bevorzugt in einem Bereich von 0,01 bis 5 Gew.-%, noch mehr bevorzugt in einem Bereich von 0,1 bis 2,5 Gew.-% und am meisten bevorzugt in einem Bereich von 0,5 bis 1,5 Gew.-%, jeweils bezogen auf das Gesamtgewicht der Paste B, beinhaltet.

Paste B beinhaltet weiterhin als Komponente (b5) wenigstens einen Komplexbildner für die Alkali-Ionen oder Erdalkali-Ionen (b4), der wenigstens zwei Ethergruppen enthält, wobei der wenigstens eine Komplexbildner (b5) vorzugsweise ausgewählt ist aus der Gruppe bestehend aus einem Podanden, einem Coronanden (= Kronenether), einem Kryptanden oder einer Mischung aus mindestens zwei davon.

Unter dem Begriff "Podanden," werden offenkettige Verbindungen verstanden, die in einer linearen oder verzweigten Kette Donoratome, beispielsweise Sauerstoffatome, Schwefelatome, Stickstoffatome oder Phosphoratome, tragen. Das bedeutet, dass die Podanden keinen preformierten Hohlraum für Kationen aufweisen. Erst bei Komplexierung von Kationen bilden sie um die Kationen Hohlräume aus. Unter "Coronanden" werden cyclische Polyether verstanden, die Ethanbrücken enthalten, die über Sauerstoffatome miteinander verbunden sind. Die Coronanden haben im Gegensatz zu den Podanden vorgebildete Hohlräume mit definierten Abmessungen. Dadurch sind Coronanden in der Lage, selektiv Kationen entsprechend ihres Ionenradius zu komplexieren, "Kryptanden" sind dagegen bicyclische und polycyclische Polyether mit ebenfalls vorgebildeten Hohlräumen.

Erfindungsgemäß bevorzugte Komplexbildner (b5) sind ausgewählt aus der Gruppe bestehend aus Benzo-12-Krone-4, Cyclohexyl-12-Krone-4, 2,3-Naphto-12-Krone-4, 6,6-Dibenzyl-12-Klone-4, 6-Dodecyl(14-Krone-4)-6-ethanoldi-ethyl-phosphat, Bis[(12-Krone-4)-methyl]-2-dodecyl-2-methyl-malonat und einer Mischung aus mindestens zwei davon. Diese Komplexbildner sind für speziell zur selektiven Komplexierung von Lithium-Ionen geeignet. Der Vorteil dieser Komplexbildner besteht darin, dass die komplexierende Kronenetherstruktur eine Ringgröße von 12 Atomen und in einem Fall von 15 Atomen hat. Dadurch können im humanen Organismus vorhandene Alkali-Ionen, wie Natrium und Kalium, als auch Erdalkali-Ionen, wie Kalzium-Ionen, nicht komplexiert werden. Dadurch wird das Risiko von eventuellen toxischen Effekten vermindert. Neben diesen kleinen Coronanden sind auch hydrophobe Derivate der 18-Krone-6, wie Benzo-18-Krone-6, Cyclohexyl--18-Krone-6 prinzipiell geeignet. Weiterhin bevorzugte Komplexbildner sind , N,N-Diheptyl-N,N',5,5-tetramethyl-3,7-dioxanonamid oder 5-Butyl-5-ethyl-N,N,-N',N'-tetracydohexyl-3,7-dioxaazelainsäurediamid.

Weiterhin ist es erfindungsgemäß bevorzugt, dass das Molverhältnis des Alkali- oder Erdalkalihalogenids (b4) zum Komplexbildner (b5) in der Paste B mindestens 1:1, besonders bevorzugt mindestens 1 : 1,5, darüber hinaus bevorzugt mindestens 1 : 1,8 und darüber hinaus noch mehr bevorzugt mindestens 1:2,0 beträgt.

Weiterhin kann die Paste B als weitere Komponente (b6) Wasser beinhalten, wobei es bevorzugt ist, dass das Masseverhältnis von Wasser (b6) zu Alkali- bzw. Erdalkalihalogenid (b4) mindestens 1:1, besonders bevorzugt mindestens 2 : 1, darüber hinaus bevorzugt mindestens 3 : 1 und darüber hinaus noch mehr bevorzugt mindestens 4 : 1 beträgt. Die Anwesenheit von Wasser in der Paste B erleichtert die Komplexierung der Kationen des Alkali- oder Erdalkalihalogenids (b4) durch den Komplexbildner (b5), so dass die komplexierten Kationen in das radikalisch polymerisierbare Monomer (b1) transportiert werden können.

Der erfindungsgemäße Kit ist dadurch gekennzeichnet, dass mindestens eine der Pasten A und B als Komponente (a3) bzw, (b3) wenigstens einen in (a1) bzw. (b1) unlöslichen Füllstoff beinhaltet. Sofern eine der beiden Pasten einen unlöslichen Füllstoff beinhaltet und die andere Paste überhaupt keinen unlöslichen Füllstoff oder einen unlöslichen Füllstoff in einer im Vergleich zur Menge in der anderen Paste vernachlässigbaren Menge, so handelt es sich um einen sogenannten "asymmetrischen" Kit. Ist hingegen der unlösliche Füllstoff in beiden Pasten in etwa vergleichbarer Menge vorhanden, so handelt es sich um einen sogenannten "symmetrischen" Kit.

Bei dem Füllstoff (a3) (im Falle der Paste A) bzw. (b3) (im Falle der Paste B) handelt es sich um einen bei Raumtemperatur festen Stoff, der in der Lage ist, die Viskosität der aus den übrigen in Paste A bzw. Paste B enthaltenen Bestandteilen zusammengesetzten Mischung zu erhöhen. Der Füllstoff (a3) bzw. (b3) soll biokompatibel sein.

Gemäß einer bevorzugten Ausführungsform ist der Füllstoff (a3) bzw, (b3) aus Polymeren, anorganischen Salzen, anorganischen Oxiden, Metallen und Metalllegierungen ausgewählt.

Der Füllstoff (a3) bzw. (b3) ist vorzugsweise partikulär. Gemäß einer besonders bevorzugten Ausführungsform weist der Füllstoff (a3) bzw. (b3) eine durchschnittliche Teilchengröße im Bereich von 10 nm bis 100 µm und besonders bevorzugt im Bereich von 100 nm bis 10 µm auf. Unter durchschnittlicher Teilchengröße wird vorliegend ein Größenbereich verstanden, der von wenigstens 90 Prozent der Teilchen eingenommen wird,

Im Rahmen der Erfindung werden unter dem Begriff Polymere sowohl Homopolymere als auch Copolymere zusammengefasst.

Bei dem als Füllstoff (a3) bzw. (b3) verwendbaren Polymer handelt es sich vorzugsweise um ein Polymer mit einer gewichtsmittleren Molmasse von wenigstens 150.000 g/mol. Die Angabe der Molmasse bezieht sich hierin auf die viskosimetrisch bestimmte Molmasse. Beispielsweise kann es sich bei dem Polymer um ein Polymer oder Copolymer eines Methacrylsäureesters handeln. Gemäß einer besonders bevorzugten Ausführungsform ist das wenigstens eine Polymer aus der Gruppe ausgewählt, die aus Polymethacrylsäuremethylester (PMMA), Polymethacrylsäureethylester (PMAE), Polymethacrylsäurepxopylester (PMAP), Polymethacrylsäureisopropylester, Poly(methylmethacrylat-co-methylacrylat) und Poly(styren-co-methylmethacrylat) besteht. Das Polymer kann jedoch ebenso aus der Gruppe ausgewählt sein, die aus Polyethylen, Polypropylen oder Polybutadien besteht. Das Polymer kann zudem vernetzt oder unvemetzt sein, wobei vernetzte Polymere besonders bevorzugt sind, Die Vernetzung erfolgt dabei vorzugsweise mit einer difunktionellen Verbindung. Die difunktionelle Verbindung kann beispielsweise aus der Gruppe ausgewählt sein, die aus Alkylenglykoldimeth-acrylaten besteht. Als Vernetzter hat sich beispielsweise Ethylenglykol-dimethacrylat bewährt.

Das als Füllstoff (a3) bzw. (b3) verwendbare anorganische Salz kann ein im radikalisch polymerisierbaren Monomer (a1) bzw. (b1) lösliches oder unlösliches Salz sein. Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Kalziumsulfat, Bariumsulfat oder Kalziumcarbonat.

Bei dem als Füllstoff (a3) bzw. (b3) verwendbaren anorganischen Oxid kann es sich vorzugsweise um ein Metalloxid handeln, Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid.

Bei dem als Füllstoff (a3) bzw. (b3) verwendbaren Metall kann es sich beispielsweise um ein Übergangsmetall handeln. Gemäß einer bevorzugten Ausführungsform ist das Metall Tantal oder Wolfram.

Die als Füllstoff (a3) bzw. (b3) verwendbare Metallegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Der Füllstoff (a3) bzw. (b3) ist in dem radikalisch polymerisierbaren Monomer (a1) bzw. (b1) unlöslich. Erfindungsgemäß ist der Füllstoff (a3) bzw. (b3) in dem radikalisch polymerisierbaren Monomer (a1) bzw. (b1) unlöslich, wenn der Füllstoff (a3) bzw. (b3) bei einer Temperatur von 25°C eine Löslichkeit im radikalisch polymerisierbaren Monomer (a1) bzw. (b1) von weniger als 50 g/l, vorzugsweise weniger als 25 g/l, mehr bevorzugt weniger als 10 g/l und noch mehr bevorzugt weniger als 5 g/l aufweist.

Erfindungsgemäß besonders bevorzugt ist es, dass das wenigstens eine in (a1) bzw. (b1) unlösliche Polymer ausgewählt ist aus der Gruppe bestehend aus vernetztem Poly(methylmethacrylat-co-methylacrylat), vernetztem Poly(methylmeth-acrylat) und einer Mischung dieser beiden Polymere.

Weiterhin kann erfindungsgemäß die Paste A, die Paste B oder die Paste A und die Paste B, besonders bevorzugt jedoch die Paste A und die Paste B, ein in (a1) bzw. (b1) lösliches Polymer (a7) bzw. (b7) beinhalten. Dieses Polymer (a7) bzw. (b7) ist erfindungsgemäß in dem polymerisierbaren Monomer, das in der Paste enthalten ist, in der auch das lösliche Polymer enthalten ist, löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer (a1) bzw. (b1) lösliche Polymer (a7) bzw. (b7) kann ein Homopolymer oder ein Copolymer sein. Bei diesem Polymer (a7) bzw. (b7) handelt es sich vorzugsweise um ein Polymer mit einer gewichtsmittleren Molmasse von wenigstens 150.000 g/mol Beispielsweise kann es sich bei dem Polymer (a7) bzw. (b7) um ein Polymer oder Copolymer eines Methacrylsäureesters handeln. Gemäß einer besonders bevorzugten Ausführungsform ist das wenigstens eine Polymer (a7) bzw. (b75) aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methyl-acrylat) und Poly(styren-co-methylmethacrylat) besteht.

Die Menge des in dem radikalisch polymerisierbaren Monomer (a1) bzw. (b1) löslichen Polymers (a7) bzw. (b7) in der Paste, die dieses Polymer enthält, hängt davon ab, ob die entsprechende Paste ein in dem radikalisch polymerisierbaren Monomer (a1) bzw, (b1) unlöslichen Füllstoff (a3) bzw. (b3) enthält. Üblicherweise liegt die Menge des in dem radikalisch polymerisierbaren Monomer (a1) bzw. (b1) löslichen Polymers (a7) bzw. (b7) in der Paste, die dieses Polymer enthält, in einem Bereich von 1 bis 85 Gew.-%, bezogen auf das Gesamtgewicht der Paste, welche dieses lösliche Polymer enthält.

Neben den vorstehend erläuterten Bestandteilen können die Pasten A und B weitere Bestandteile aufweisen. Diese weiteren Bestandteile können jeweils entweder in Paste A, in Paste B oder in der Paste A und der Paste B enthalten sein.

Gemäß einer bevorzugten Ausführungsform ist in wenigstens einer der Pasten A und B wenigstens ein Röntgenopaker enthalten. Beim Röntgenopaker kann es sich um einen fachüblichen Röntgenopaker handeln. Geeignete Röntgenopaker können in dem radikalisch polymerisierbaren Monomer (a1) oder dem radikalisch polymerisierbaren Monomer (b1) löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht.

Gemäß einer weiteren bevorzugten Ausführungsform ist in wenigstens einer der Pasten A und B wenigstens ein Farbstoff enthalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer (a1) oder in dem wenigstens einen radikalisch polymerisierbaren Monomer (a2) löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform ist in wenigstens einer der Pasten A und B wenigstens ein pharmazeutischer Wirkstoff enthalten. Der wenigstens eine pharmazeutische Wirkstoff kann in wenigstens einer der Pasten A und B in gelöster oder suspendierter Form enthalten sein.

Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum.

Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein die aus Gentamicinsulfat, Gentamicinhydrochlorid, Amikacinsulfat, Amikacinhydrochlorid, Tobramycinsulfat, Tobramycinhydrochlorid, Clindamycin-hydrochlorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausfühnmgsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht.

Das wenigstens eine Cytostatikum, ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

Gemäß einer weiteren bevorzugten Ausführungsform ist in wenigstens einer der Pasten A und B wenigstens ein biokompatibles Elastomer enthalten. Das biokompatible Elastomer ist vorzugsweise partikulär. Vorzugsweise ist das biokompatible Elastomer in dem wenigstens einen radikalisch polymerisierbaren Monomer (a1) oder in dem wenigstens einen radikalisch polymerisierbaren Monomer (b1) löslich. Als besonders geeignet hat sich die Verwendung von Polybutadien als biokompatibles Elastomer erwiesen.

Gemäß einer weiteren bevorzugten Ausführungsform enthält wenigstens eine der Pasten A und B wenigstens ein Monomer mit Haftgruppen. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform ist in wenigstens einer der Pasten A und B wenigstens ein Stabilisator enthalten. Der Stabilisator soll geeignet sein, eine Spontanpolymerisation der in den Pasten A und B enthaltenen radikalisch polymerisierbaren Monomere (a1) bzw. (b1) zu verhindern. Ferner soll der Stabilisator keine störenden Wechselwirkungen mit den anderen in den Pasten enthaltenen Bestandteilen zeigen. Derartige Stabilisatoren sind aus dem Stand der Technik bekannt. Gemäß einer bevorzugten Ausführungsform handelt es sich bei dem Stabilisator um 2,5-Di-tert-butyl-4-methylphenol und/oder 2,6-Di-tert-butyl-phenol.

Gemäß einer ersten besonderen Ausgestaltung des erfindungsgemäßen Kits handelt es bei dem Kit um einen "asymmetrischen" Kit In diesem Zusammenhang ist es bevorzugt, dass die Paste A 20 bis 70 Gew.-%, besonders bevorzugt 25 bis 60 Gew.-%, noch mehr bevorzugt 30 bis 55 Gew.-% und am meisten bevorzugt 34 bis 47 Gew.-%, jeweils bezogen auf das Gesamtgewicht der Paste A, des in (a1) unlöslichen Füllstoffs (a3) und Paste B weniger als 5 Gew.-%, besonders bevorzugt weniger als 1 Gew.-%, noch mehr bevorzugt weniger als 0,1 Gew.-% und darüber hinaus bevorzugt weniger als 0,01 Gew.-%, jeweils bezogen auf das Gesamtgewicht der Paste B, des in (b1) unlöslichen Füllstoffs (b3) enthält, wobei es am meisten bevorzugt ist, dass die Paste B überhaupt kein in (b1) unlöslichen Füllstoff (b3) enthält.

Weiterhin ist es im Zusammenhang mit dieser ersten besonderen Ausgestaltung des erfindungsgemäßen Kits bevorzugt, dass Paste A ein in (a1) lösliches Polymer (a7) in einer Menge in einem Bereich von 1 bis 25 Gew.-%, besonders bevorzugt in einem Bereich von 2 bis 20 Gew.-%, noch mehr bevorzugt in einem Bereich von 2 bis 18 Gew.-% und am meisten bevorzugt in einem Bereich von 3 bis 16 Gew.-%, jeweils bezogen auf das Gesamtgewicht der Paste A, und Paste B ein in (b1) lösliches Polymer (b7) in einer Menge in einem Bereich von 25 bis 85 Gew.-%, besonders bevorzugt in einem Bereich von 35 bis 85 Gew.-%, noch mehr bevorzugt in einem Bereich von 40 bis 80 Gew.-% und am meisten bevorzugt in einem Bereich von 50 bis 75 Gew.-%, jeweils bezogen auf das Gesamtgewicht der Paste B, beinhalten,

Weiterhin ist es im Zusammenhang mit dieser ersten besonderen Ausgestaltung des erfindungsgemäßen Kits bevorzugt, dass das Gewichtsverhältnis von in (b1) unlöslichem Füllstoff (b3) zu dem wenigstens einen in (b1) löslichen Polymer (b7) höchstens 0,2, besonders bevorzugt höchstens 0,15, noch mehr bevorzugt höchstens 0,1, nicht mehr bevorzugt höchstens 0,05, besonders bevorzugt höchstens 0,02 und ganz besonders bevorzugt 0 beträgt.

Gemäß einer zweiten besonderen Ausgestaltung des erfindungsgemäßen Kits handelt es bei dem Kit um einen "symmetrischen" Kit. In diesem Zusammenhang ist es bevorzugt, dass die Paste A 15 bis 85 Gew.-%, besonders bevorzugt 15 bis 80 Gew.-% und noch mehr bevorzugt 20 bis 75 Gew.-%, jeweils bezogen auf das Gesamtgewicht der Paste A, des in (a1) unlöslichen Füllstoffs (a3) und Paste B 15 bis 85 Gew.-%, besonders bevorzugt 15 bis 80 Gew.-% und noch mehr bevorzugt 20 bis 75 Gew.-%, jeweils bezogen auf das Gesamtgewicht der Paste B, des in (b1) unlöslichen Füllstoffs (b3) beinhalten.

Weiterhin ist es im Zusammenhang mit dieser zweiten besonderen Ausgestaltung des erfindungsgemäßen Kits bevorzugt, dass Paste A ein in (a1) lösliches Polymer (a7) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, jeweils bezogen auf das Gesamtgewicht der Paste A, und/oder Paste B ein in (b1) lösliches Polymer (b7) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, bezogen auf das Gesamtgewicht der Paste B, beinhalten.

Erfindungsgemäß dient der Kit, der wenigstens die Pasten A und B enthält, zur Herstellung von Knochenzement.

Dazu werden die wenigstens zwei Pasten A und B miteinander vermischt, wobei wiederum eine Paste, Paste C, erhalten wird.

Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B.

Das Mischen kann mit üblichen Mischgeräten, beispielsweise einem statischen Mischer oder einem dynamischen Mischer erfolgen.

Das Mischen kann unter Vakuum erfolgen. Die Verwendung des erfindungsgemäßen Initiatorsystems erlaubt jedoch auch das vakuumfreie Mischen der Paste A und B ohne Beeinträchtigung der Eigenschaften des Knochenzements.

Nach dem Vermischen der Pasten des Kits ist die letztlich erhaltene Paste C nach der Norm ISO 5833 klebfrei und kann sofort verarbeitet werden.

Der aus der Paste C durch Aushärtung entstehende Knochenzement erreicht etwa sechs bis acht Minuten nach dem Vennischen der im Kit enthaltenen Pasten eine hohe Festigkeit.

Der erfindungsgemäße Kit kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacem und zur Herstellung von Trägermaterialien für die lokale Antibiotika-Therapie verwendet werden.

Unter dem Begriff *"Spacer"* werden dabei erfindungsgemäß Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bobnenförmige Körper ausgebildet sein. Daneben ist es auch möglich, stabförmige oder scheibenförmige Trägermaterialien herzustellen, die den aus dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.

Die vorstehend beschriebenen erfindungsgemäßen Verwendungen von Knochenzement sind aus der Literatur bekannt und dort mannigfach beschrieben.

Erfindungsgemäß wird der Kit für die vorstehend beschrieben Verwendungen eingesetzt, indem vorzugsweise aus den im Kit enthaltenen Pasten durch Vermischen eine Paste hergestellt wird, die analog zu den aus dem Stand der Technik bekannten Pasten für die beschriebenen Verwendungen eingesetzt wird.

Einen Beitrag zur Lösung der eingangs genannten Aufgaben leistet weiterhin ein Initiatorsystem, beinhaltend
(i1) mindestens ein Barbitursäurederivat,
(i2) mindestens ein Schwermetallsalz,
(i3) mindestens ein Alkali- oder Erdalkalihalogenid,
(i4) mindestens einen Komplexbildner für Alkali- oder Erdalkali-Ionen, der wenigstens zwei Ethergruppen enthält, sowie
(i5) gegebenenfalls Wasser.

Als Barbitursäurederivat (i1), als Schermetallsalz (i2), als Alkali- oder Erdalkalihalogenid (i3) und als Komplexbildner für Alkali- oder Erdalkali-Ionen (i4) sind dabei diejenigen Verbindungen bevorzugt, die bereits eingangs im Zusammenhang mit dem erfindungsgemäßen Kit als bevorzugte Barbitursäurederivate (a2), als Schermetallsalz (b2), Alkali- oder Erdalkalihalogenide (b4) und Komplexbildner für Alkali- oder Erdalkali-Ionen (b5) genannt wurden.

Einen Beitrag zur Lösung der eingangs genannten Aufgaben leistet auch eine polymerisierbare Zusammensetzung, beinhaltend wenigstens ein radikalisch polymerisierbares Monomer, vorzugsweise ein Methacrylat-Monomer, und ein erfindungsgemäßes Initiatorsystem. Eine solche polymerisierbare Zusammensetzung kann beispielsweise durch das Vermischen der Pasten A und B des erfindungsgemäßen Kits erhalten werden.

Einen Beitrag zur Lösung der eingangs genannten Aufgaben leistet auch ein Formkörper, erhalten durch die Polymerisation der erfindungsgemäßen polymerisierbaren Zusammensetzung oder durch die Polymerisation einer Paste, erhältlich durch das Vermischen der Paste A und der Paste B des erfindungsgemäßen Kits. Formkörper im Sinne der vorliegenden Erfindung können jedwede dreidimensionalen Körper sein, insbesondere die vorstehend beschriebenen "Spacer".

Die Erfindung wird durch die nachstehend beschriebenen Beispiele erläutert, die jedoch die Erfindung nicht einschränken.

### BEISPIELE

### Beispiele 1 bis 4 (erfindungsgemäß)

Die Pasten A der Beispiele 1-4 wurden durch einfaches Vermischen der Komponenten hergestellt. Die gebildeten Pasten wurden dann über Nacht bei Raumtemperatur gelagert.

| Rohstoff | Zusammensetzung der Paste A | |
|---|---|---|
| | Beispiel 1 | Beispiele 2-4 |
| 1-Cyclohexyl-5-ethyl-barbitursäure (a2) | 2,0 g | 2,0 g |
| MMA(a1) | 19,0 g | 19,0 g |
| Methacrylamid | 0,4 g | 0,4 g |
| Ethylenglykoldimethacrylat | 0,1 g | 0,1 g |
| Lösliches PMMA (a7) | 6,0 g | 6,0 g |
| Vernetztes PMMA (a3) | 15,0 g | 12,7 g |
| 2,6-di-tert.-Butyl-4-methyl-phenol | 20 mg | 20 mg |
| - | - | - |
| Gentamycinsulfat (Aktivitätskoeffizient 628) | - | 2,3g |

Die Pasten B wurden in der Weise hergestellt, dass zuerst das Lithiumchlorid, das Wasser und MMA eingewogen wurden. Das Gemisch wurde bei Raumtemperatur solange gerührt, bis die wässrige Phase visuell nicht mehr erkennbar war. Danach wurden alle weiteren Komponenten dazugegeben und das Gemisch wurde durch Rühren homogenisiert. Die entstanden Pasten wurden dann bei Raumtemperatur über Nacht gelagert.

| Rohstoff | Zusammensetzung der Paste B | | | |
|---|---|---|---|---|
| | Beispiel 1 | Beispiel 2 | Beispiel 3 | Beispiel 4 |
| Lithiumchlorid (b4) | 40 mg | 40 mg | 40 mg | 40 mg |
| Benzo-12-Krone-4 (b5) | 317 mg | 317 mg | 317 mg | 317 mg |
| Dest. Wasser (b6) | 80 mg | 80 mg | 80 mg | 80 mg |
| 2,6-di-tert.-Butyl-4-methylphenol | 35-mg | 35- mg | 35- mg | 35- mg |
| MMA (b1) | 21,0 g | -21,0 g | 21,00-g | 21,0-g |
| Lösliches PMMA (b7) | 17,0 g | 17,0 g | 17,0 g | 17,0 g |
| Zirkoniumdioxid | 5,0 g | 5,0 g | 5,0 g | 5,0 g |
| Kupfer(II)hydroxid (b2) | 6,0 mg | 4,0 mg | 6,0 mg | 8,0 mg |

Die Pasten A und B der Beispiele 1-4 wurden jeweils im Gewichtverhältnis 1 : 1 miteinander gemischt (Paste A aus Beispiel 1 mit Paste B aus Beispiel 1 usw.). Es entstanden sofort klebfreie Pasten, die nach wenigen Minuten aushärteten.

Mit den aus den Pasten A und B der Beispiele 1-4 hergestellten gemischten Pasten wurden streifenförmige Prüfkörper mit den Abmessungen (75 mm × 10 mm × 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden 24 Stunden bei 37°C in Wasser gelagert. Danach wurden die 4-Punkt-Biegefestigkeit, das Biegemodul und die Druckfestigkeit der Prüfkörper mit einer Zwick-Universalprüfmaschine ermittelt. Weiterhin wurden streifenförmige Prüfkörper der Abmessungen (20 mm × 10 mm × 3,3 mm) hergestellt und 24 Stunden bei 37° in Wasser gelagert. Danach wurde die Dynstat-Biegefestigkeit und die Dynstat-Schlagzähigkeit dieser Prüfkörper mit Hilfe einer Dynstat-Prüfmaschine bestimmt.

| | Beispiele | | | |
|---|---|---|---|---|
| | 1 | 2 | 3 | 4 |
| 4-Punkt-Biegefestigkeit [MPA] | 70,0 ± 1,7 | 59,2 ± 2,2 | 59,9 ± 2,5 | 59,6 ± 1,9 |
| Biegemodul [MPA] | 2.615 ± 59 | 2.347 ± 56 | 2.377 ± 56 | 2,204 ± 115 |
| Druckfestigkeit [MPA] | 95,3 ± 3,1 | 80,6 ± 2,5 | 82,0 ± 1,7 | 83,3 ± 3,1 |
| Dynstat-Biegefestigkeit [MPA] | 94,6 ± 4,3 | 70,1 ± 3,2 | 76,0 ± 3,5 | 74,1 ± 2,5 |
| Dynstat-Schlagzähigkeit [kJ/m²] | 5,2 ± 0,3 | 3,5 ± 0,3 | 3,9 ± 0,4 | 4,0 ± 0,3 |

### Beispiele 5-11 (erfindungsgemäß)

Es wurden Pasten A und B gemäß Beispiel 1 hergestellt, wobei anstelle des Komplexbildners Benzo-12-Krone-4 der Komplexbildner Cyclohexyl-12-Krone-4, 2,3-Naphto-12-Krone-4 (Beispiel 5), 6,6-Dibenzyl-12-Krone-4, 6-Dodecyl(14-Krone-4)-6-ethanoldi-ethyl-phosphat (Beispiel 6), Bis[(12-Krone-4)-methyl]-2-dodecyl-2-methyl-malonat (Beispiel 7), Benzo-18-Krone-6 (Beispiel 8), Cyclohexyl-18-Krone-6 (Beispiel 9), N,N-Diheptyl-N,N',5,5-tetramethyl-3,7-dioxanonamid (Beispiel 10) oder 5-Butyl-5-ethyl-N,N,-N',N'-tetracyclohexyl-3,7-dioxaazelain-säurediamid (Beispiel 11) in der gleichen molaren Menge in der Paste B eingesetzt wurde, in der auch Benzo-12-Krone-4 im Beispiel 1 in der Paste B enthalten war.

Die Pasten A und B der Beispiele 5-11 wurden jeweils wieder im Gewichtverhältnis 1 : 1 miteinander gemischt. Es entstanden sofort klebfreie Pasten, die nach wenigen Minuten aushärteten.

### Vergleichsbeispiel (nicht erfindungsgemäß)

Es wurden Pasten A und B aus Beispiel 1 hergestellt, wobei Paste B keinen Komplexbildner enthielt. Es zeigte sich, dass bei der Vermischung von Methylmethacrylat, Wasser und Lithiumchlorid ohne Zusatz des Komplexbildners zwei Phasenentstanden, wobei sich die Hauptmenge des Lithiumchlorids in der wässrigen Phase sammelte. Die wässrige Phase blieb auch nach mehrtägigem Rühren bestehen. Es wurde trotzdem eine Paste B hergestellt. Jedoch war die Polymerisation nach dem Mischen der Paste A mit der Paste B stark verzögert, wobei das Ausmaß dieser Verzögerung unter anderem vom Wassergehalt in der Paste B abhängig war.

## Patentansprüche

1. Kit, aufweisend eine Paste A und eine Paste B,
wobei
(a) Paste A
(a1) wenigstens ein radikalisch polymerisierbares Monomer, und
(a2) als Polymerisationsinitiator wenigstens ein Barbitursäurederivat beinhaltet;
(b) Paste B
(b1) wenigstens ein radikalisch polymerisierbares Monomer,
(b2) als Polymerisationsbeschleuniger wenigstens eine Schwermetallverbindung, die aus der Gruppe bestehend aus Schwermetallsalzen und Schwermetallkomplexen ausgewählt ist,
(b4) wenigstens ein Alkali- oder Erdalkalihalogenid, und
(b5) wenigstens einen Komplexbildner für die Alkali-Ionen oder Erdalkali-Ionen (b4), der wenigstens zwei Ethergruppen enthält, beinhaltet;
und wobei mindestens eine der Pasten A und B als Komponente (a3) bzw. (b3) wenigstens einen in (a1) bzw. (b1) unlöslichen Füllstoff beinhaltet.

2. Kit nach Anspruch 1, wobei das wenigstens eine radikalisch polymerisierbare Monomer (a1) bzw. (b1) ein Methacrylat-Monomer ist.

3. Kit nach Anspruch 1 oder 2, wobei die Paste A und die Paste B das wenigstens eine radikalisch polymerisierbare Monomer (a1) bzw. (b1) in einer Menge in einem Bereich von 15 bis 85 Gew.-%, jeweils bezogen auf das Gesamtgewicht der Paste A bzw. der Paste B, beinhalten.

4. Kit nach einem der vorhergehenden Ansprüche, wobei das wenigstens eine Barbitursäurederivat (a2) ausgewählt ist aus der Gruppe bestehend aus 1-Cyclohexyl-ethylbarbitursäure, 1-Phenyl-5-ethyl-barbitursäure und 1,3,5-Trimethyl-barbitursäure.

5. Kit nach einem der vorhergehenden Ansprüche, wobei die Paste A das wenigstens eine Barbitursäurederivat (a2) in einer Menge in einem Bereich von 0,1 bis 10 Gew.-%, jeweils bezogen auf das Gesamtgewicht der Paste A, beinhaltet.

6. Kit nach einem der vorhergehenden Ansprüche, wobei die wenigstens eine Schwermetallverbindung (b2) ausgewählt ist aus der Gruppe bestehend aus Kupfer(II)-hydroxid, Cobalt(II)-hydroxid- und basisches Kupfer(II)-carbonat.

7. Kit nach einem der vorhergehenden Ansprüche, wobei die Paste B die Schwermetallverbindung (b2) in einer Menge in einem Bereich von 0,0005 bis 0,5 Gew.-%, bezogen auf das Gesamtgewicht der Paste B, beinhaltet.

8. Kit nach einem der vorhergehenden Ansprüche, wobei das wenigstens eine Alkali- oder Erdalkalihalogenid (b4) ein Alkalichlorid, ein Erdalkalichlorid oder eine Mischung aus mindestens zwei davon ist.

9. Kit nach einem der vorhergehenden Ansprüche, wobei das wenigstens eine Alkalihalogenid (b4) Lithiumchlorid ist.

10. Kit nach einem der vorhergehenden Ansprüche, wobei, Paste B das mindestens eine Alkali- oder Erdalkalihalogenid (b4) in einer Menge in einem Bereich von 0,001 bis 7,5 Gew.-%, bezogen auf das Gesamtgewicht der B, beinhaltet.

11. Kit nach einem der vorhergehenden Ansprüche, wobei der wenigstens eine Komplexbildner (b5) ausgewählt ist aus der Gruppe bestehend aus einem Podanden, einem Coronanden -, einem Kryptanden oder einer Mischung aus mindestens zwei davon.

12. Kit nach einem der vorhergehenden Ansprüche, wobei der wenigstens eine Komplexbildner (b5) ausgewählt ist aus der Gruppe bestehend aus Benzo-12-Krone-4, Cyclohexyl-12-Krone-4, 2,3-Naphto-12-Krone-4, 6,6-Dibenzyl-12-Krone-4, 6-Dodecyl(14-Krone-4)-6-ethanoldi-ethyl-phosphat, Bis[(12-Krone-4)-methyl]-2-dodecyl-2-methyl-malonat, N,N-Diheptyl-N,N',5,5-tetramethyl-3,7-dioxanonamide, 5-Butyl-5-ethyl-N,N,-N',N'-tetracyclohexyl-3,7-dioaazelaicdiamid und einer Mischung aus mindestens zwei davon.

13. Kit nach einem der vorhergehenden Ansprüche, wobei das Molverhältnis des Alkali- oder Erdalkalihalogenids (b4) zum Komplexbildner (b5) in der Paste B mindestens 1 : 1 beträgt.

14. Kit nach einem der vorhergehenden Ansprüche, wobei der wenigstens eine in (a1) bzw. (b1) unlösliche Füllstoff (a3) bzw. (b3) ein partikuläres Polymer ist.

15. Kit nach einem der vorhergehenden Ansprüche, wobei der wenigstens eine in (a1) bzw. (b1) unlösliche Füllstoff (a3) bzw. (b3) ein unlösliches Polymer ausgewählt aus der Gruppe bestehend aus vernetztem Poly(methylmethacrylat-co-methylacrylat), vernetztem Poly(methylmetha-crylat) und einer Mischung dieser beiden Polymere ist.

16. Kit nach einem der vorhergehenden Ansprüche, wobei diejenige Paste B als weitere Komponente (b6) Wasser beinhaltet.

17. Kit nach Anspruch 16, wobei das Masseverhältnis von Wasser (b6) zu Alkali- bzw. Erdalkalihalogenid (b4) mindestens 1 : 1 beträgt.

18. Kit nach einem der vorhergehenden Ansprüche, wobei Paste A, Paste B oder Paste A und Paste B ein in (a1) bzw. (b1) lösliches Polymer (a7) bzw. (b7) beinhalten.

19. Kit nach Anspruch 18, wobei das in (a1) bzw. (b1) lösliche Polymer (a7) bzw. (b7) ausgewählt ist aus der Gruppe bestehend aus Poly(methacrylsäure-methylester), Poly(methacrylsäureethylester), Poly-(methylmethacrylsäurepropylester), Poly(methacrylsäureisopropylester), Poly(methylmeth-acrylat-co-methylacrylat) und Poly(styrol-co-methyl-methacrylat).

20. Verwendung eines Kits nach einem der vorhergehenden Ansprüche zur Herstellung einer Paste zur Herstellung von Spacern oder zur Herstellung von Trägermaterialien für die lokale Antibiotika-Therapie.

## Claims

1. Kit, comprising a paste A and a paste B,
whereby
(a) paste A contains
(a1) at least one monomer for radical polymerisation, and
(a2) at least one barbituric acid derivative as polymerisation initiator;
(b) paste B contains
(b1) at least one monomer for radical polymerisation,
(b2) at least one heavy metal compound as polymerisation accelerator selected from the group consisting of heavy metal salts and heavy metal complexes,
(b4) at least one alkali or alkaline earth halogenide; and
(b5) at least one complex-forming agent for the alkali ions or alkaline earth ions (b4) that contains at least two ether groups;
and whereby at least one of the pastes A and B contains, as component (a3) and/or (b3), at least one filling agent that is insoluble in (a1) and/or (b1).

2. Kit according to claim 1, whereby the at least one monomer for radical polymerisation (a1) and/or (b1) is a methacrylate monomer.

3. Kit according to claim 1 or 2, whereby paste A and paste B contain the at least one monomer for radical polymerisation (a1) and/or (b1) in an amount in the range of 15 to 85% by weight, each relative to the total weight of paste A and/or paste B.

4. Kit according to any one of the preceding claims, whereby the at least one barbituric acid derivative (a2) is selected from the group consisting of 1-cyclohexyl-ethyl-barbituric acid, 1-phenyl-5-ethyl-barbituric acid and 1,3,5-trimethyl-barbituric acid.

5. Kit according to any one of the preceding claims, whereby paste A contains the at least one barbituric acid derivative (a2) in an amount in the range of 0.1 to 10% by weight, each relative to the total weight of paste A.

6. Kit according to any one of the preceding claims, whereby the at least one heavy metal compound (b2) is selected from the group consisting of copper(II) hydroxide, cobalt(II) hydroxide and basic copper(II) carbonate.

7. Kit according to any one of the preceding claims, whereby paste B contains the heavy metal compound (b2) in an amount in the range of 0.0005 to 0.5% by weight, relative to the total weight of paste B.

8. Kit according to any one of the preceding claims, whereby the at least one alkali or alkaline earth halogenide (b4) is an alkali chloride, an alkaline earth chloride or a mixture of at least two thereof.

9. Kit according to any one of the preceding claims, whereby the at least one alkali halogenide (b4) is lithium chloride.

10. Kit according to any one of the preceding claims, whereby paste B contains the at least one alkali halogenide or alkaline earth halogenide (b4) in an amount in the range of 0.001 to 7.5% by weight, relative to the total weight of paste B.

11. Kit according to any one of the preceding claims, whereby the at least one complex-forming agent (b5) is selected from the group consisting of a podand, a coronand, a cryptand or a mixture of at least two thereof.

12. Kit according to any one of the preceding claims, whereby the at least one complex-forming agent (b5) is selected from the group consisting benzo-12-crown-4, cyclohexyl-12-crown-4, 2,3-naphto-12-crown-4, 6,6-dibenzyl-12-crown-4, 6-dodecyl(14-crown-4)-6-ethanoldi-ethyl-phosphate, bis[(12-crown-4)-methyl]-2-dodecyl-2-methylmalonate, N,N-diheptyl-N,N',5,5-tetramethyl-3,7-dioxanonamide, 5-butyl-5-ethyl-N,N-N',N'-tetracyclohexyl-3,7-dioaazelaicdiamide, and a mixture of at least two thereof.

13. Kit according to any one of the preceding claims, whereby the molar ratio of the alkali halogenide or alkaline earth halogenide (b4) and the complex-forming agent (b5) in paste B is at least 1:1.

14. Kit according to any one of the preceding claims, whereby the at least one filling agent (a3) and/or (b3) that is insoluble in (a1) and/or (b1) is a particulate polymer.

15. Kit according to any one of the preceding claims, whereby the at least one filling agent (a3) and/or (b3) that is insoluble in (a1) and/or (b1) is an insoluble polymer selected from the group consisting of cross-linked poly(methyl methacrylate-co-methyl acrylate), cross-linked poly(methyl methacrylate) and a mixture of these two polymers.

16. Kit according to any one of the preceding claims, whereby paste B contains water as further component (b6).

17. Kit according to claim 16, whereby the mass ratio of water (b6) and alkaline halogenide and/or alkaline earth halogenide (b4) is at least 1 : 1.

18. Kit according to any one of the preceding claims, whereby paste A, paste B or paste A and paste B contain a polymer (a7) and/or (b7) that is soluble in (a1) and/or (b1).

19. Kit according to claim 18, whereby the polymer (a7) and/or (b7) that is soluble in (a1) and/or (b1) is selected from the group consisting of poly(methacrylic acid methyl ester), poly(methacrylic acid ethyl ester), poly(methylmethacrylic acid propyl ester), poly(methacrylic acid isopropyl ester), poly(methylmethacrylate-co-methylacrylate), and poly(styrene-co-methylmethacrylate).

20. Use of a kit according to any one of the preceding claims for the production of a paste for the production of spacers or for the production of carrier materials for local antibiotics therapy.

## Revendications

1. Kit présentant une pâte A et une pâte B,
dans lequel
(a) la pâte A
(a1) contient au moins un monomère polymérisable par voie radicalaire et
(a2) au moins un dérivé d'acide barbiturique en tant qu'initiateur de polymérisation ;
(b) la pâte B
(b1) contient au moins un monomère polymérisable par voie radicalaire,
(b2) au moins un composé de métal lourd en tant qu'accélérateur de polymérisation qui est sélectionné parmi le groupe constitué de sels de métal lourd et de complexes de métal lourd,
(b4) au moins un halogénure alcalin ou alcalino-terreux, et
(b5) au moins un agent complexant pour les ions alcalins ou ions alcalino-terreux (b4) qui contient au moins deux groupes éther ;
et dans lequel au moins une des pâtes A et B contient en tant que composant (a3) ou (b3) au moins une charge insoluble dans (a1) ou (b1).

2. Kit selon la revendication 1, dans lequel l'au moins un monomère polymérisable par voie radicalaire (a1) ou (b1) est un monomère de méthacrylate.

3. Kit selon la revendication 1 ou 2, dans lequel la pâte A et la pâte B contiennent l'au moins un monomère polymérisable par voie radicalaire (a1) ou (b1) en une quantité dans une plage de 15 à 85 % en poids, à chaque fois par rapport au poids total de la pâte A ou de la pâte B.

4. Kit selon une des revendications précédentes, dans lequel l'au moins un dérivé d'acide barbiturique (a2) est sélectionné parmi le groupe constitué de l'acide 1-cyclohexyl-éthyl-barbiturique, l'acide 1-phényl-5-éthyl-barbiturique et l'acide 1,3,5-triméthyl-barbiturique.

5. Kit selon une des revendications précédentes, dans lequel la pâte A contient l'au moins un dérivé d'acide barbiturique (a2) en une quantité dans une plage de 0,1 à 10 % en poids, à chaque fois par rapport au poids total de la pâte A.

6. Kit selon une des revendications précédentes, dans lequel l'au moins un composé de métal lourd (b2) est sélectionné parmi le groupe constitué de l'hydroxyde de cuivre(II), l'hydroxyde de cobalt(II) et le carbonate de cuivre(II) basique.

7. Kit selon une des revendications précédentes, dans lequel la pâte B contient le composé de métal lourd (b2) en une quantité dans une plage de 0,0005 à 0,5 % en poids, par rapport au poids total de la pâte B.

8. Kit selon une des revendications précédentes, dans lequel l'au moins un halogénure alcalin ou alcalino-terreux (b4) est un chlorure alcalin, un chlorure alcalino-terreux ou un mélange d'au moins deux de ceux-ci.

9. Kit selon une des revendications précédentes, dans lequel l'au moins un halogénure alcalin (b4) est le chlorure de lithium.

10. Kit selon une des revendications précédentes, dans lequel la pâte B contient l'au moins un halogénure alcalin ou alcalino-terreux (b4) en une quantité dans une plage de 0,001 à 7,5 % en poids, par rapport au poids total de la pâte B.

11. Kit selon une des revendications précédentes, dans lequel l'au moins un agent complexant (b5) est sélectionné parmi le groupe constitué d'un podand, d'un coronand, d'un cryptand ou d'un mélange d'au moins deux de ceux-ci.

12. Kit selon une des revendications précédentes, dans lequel l'au moins un agent complexant (b5) est sélectionné parmi le groupe constitué du benzo-12-couronne-4, cyclohexyl-12-couronne-4, 2,3-naphto-12-couronne-4, 6,6-dibenzeyl-12-couronne-4, 6-dodécyl(14-couronne-4)-6-éthanoldi-éthyl-phosphate, bis[(12-couronne-4)-méthyl]-2-dodécyl-2-méthyl-malonate, N,N-diheptyl-N,N',5,5-tétraméthyl-3,7-dioxanonamide, diamide 5-butyl-5-éthyl-N,N,N',N'-tétracyclohexyl-3,7-dioazélaïque et d'un mélange d'au moins deux de ceux-ci.

13. Kit selon une des revendications précédentes, dans lequel le rapport molaire de l'halogénure alcalin ou alcalino-terreux (b4) sur l'agent complexant (b5) dans la pâte B se monte à au moins 1/1.

14. Kit selon une des revendications précédentes, dans lequel l'au moins une charge (a3) ou (b3) insoluble dans (a1) ou (b1) est un polymère particulaire.

15. Kit selon une des revendications précédentes, dans lequel l'au moins une charge (a3) ou (b3) insoluble dans (a1) ou (b1) est un polymère insoluble sélectionné parmi le groupe constitué du poly(méthylméthacrylate-co-méthylacrylate) réticulé, poly(méthylmétha-crylate) réticulé et d'un mélange de ces deux polymères.

16. Kit selon une des revendications précédentes, dans lequel la pâte B contient de l'eau en tant qu'autre composant (b6).

17. Kit selon la revendication 16, dans lequel le rapport massique de l'eau (b6) sur l'halogénure alcalin ou alcalino-terreux (b4) se monte à au moins 1/1.

18. Kit selon une des revendications précédentes, dans lequel la pâte A, la pâte B ou la pâte A et la pâte B contiennent un polymère (a7) ou (b7) soluble dans (a1) ou (b1).

19. Kit selon la revendication 18, dans lequel le polymère (a7) ou (b7) soluble dans (a1) ou (b1) est sélectionné parmi le groupe constitué de l'ester méthylique d'acide poly(méthacrylique), l'ester éthylique d'acide poly(méthacrylique), l'ester propylique d'acide poly-(méthylméthacrylique), l'ester isopropylique d'acide poly(méthacrylique), le poly(méthylméth-acrylate-co-méthylacrylate) et le poly(styrène-co-méthyl-méthacrylate).

20. Utilisation d'un kit selon une des revendications précédentes pour la fabrication d'une pâte pour la fabrication d'écarteurs ou pour la fabrication de matériaux de support pour l'antibiothérapie locale.
